# EUROPÄISCHE PATENTSCHRIFT

(11) **EP 0 829 721 B1**
(45) Veröffentlichungstag und Bekanntmachung des Hinweises auf die Patenterteilung: **28.08.2002**
(21) Anmeldenummer: 97115963.7
(22) Anmeldetag: 13.09.1997
(51) Int. Cl.: G01N 33/536, G01N 33/96, G01N 33/74

(54) **Stabilisierung von Standardlösungen durch Konjugate**
Stabilization of standard solutions by conjugates
Stabilisation de solutions standard par des conjugués

(30) Priorität: 17.09.1996 DE 19637803
(43) Veröffentlichungstag der Anmeldung: 18.03.1998
(73) Patentinhaber: Roche Diagnostics GmbH, 68298 Mannheim (DE)
(72) Erfinder: Unkrig, Volker, Dr., 68526 Ladenburg (DE)

(56) Entgegenhaltungen:
- EP-A- 0 650 053
- EP-A- 0 780 687

## Beschreibung

Die vorliegende Erfindung betrifft ein Verfahren zur Herstellung von Standardlösungen, die zur Kalibrierung von Meßsystemen eingesetzt werden, mit denen biologische Substanzen nachgewiesen werden können. Außerdem betrifft die Erfindung die Verwendung eines Konjugates zur Stabilisierung eines Analyten in Lösung. Des weiteren betrifft die Erfindung eine Standardlösung bestehend aus einem Komplex aus Analyt und einem Konjugat, sowie ein Verfahren zum Nachweis eines Analyten. Die Standardlösungen werden auch als Eichstandards, Eichlösungen oder Kalibratoren bezeichnet.

Immunologische Nachweisverfahren haben in der in vitro Diagnostik eine große Bedeutung erlangt. Sie zeichnen sich durch eine hohe Spezifität und Sensitivität aus, so daß sie auch für den Nachweis von niedrig konzentrierten Analyten in biologischen Flüssigkeiten gut geeignet sind. Viele klinisch relevante Substanzen wie Metabolite, Hormone, Antikörper, Antigene etc. können mit Hilfe solcher Immunoassays bestimmt werden. Diese Nachweisverfahren beruhen auf der immunologischen Wechselwirkung zwischen dem nachzuweisenden Analyten und mindestens einem Bindungspartner.

Ein Klassiker unter den Immunoassays ist der Sandwich-Assay, bei dem der Analyt zwischen zwei spezifischen Antikörpern sandwichartig gebunden wird (The Immunoassay Handbook, Herausg. D. Wild, The Macmillan Press Ltd., 1994, Kapitel "Immunoassay design", S. 15-19). Einer der beiden Antikörper ist direkt an die Festphase gebunden oder ist mit Strukturen gekoppelt, die seine Bindung an einer Festphase ermöglichen. Eine gebräuchliche Struktur ist das Molekül Biotin, das an das Protein Streptavidin sehr schnell und fest binden kann (The Immunoassay Handbook, Herausg. D. Wild, The Macmillan Press Ltd., 1994, Kapitel "Immunoassay design", S. 76-77).

Der andere Antikörper ist mit einer Markierung versehen, die ein Signal liefert, das wiederum die Detektion des ausgebildeten Sandwich ermöglicht. Die Detektion kann je nach den Eigenschaften der Markierung über die Messung von Absorption, Fluoreszenz, Lumineszenz, Chemilumineszenz, Radioaktivität etc. erfolgen.

Um das im Immunoassay erzeugte Signal mit der Konzentration des Analyten korrelieren zu können, muß der Test zuvor mit Lösungen, die eine bekannte Konzentration an Analyt bzw. Analytanalogon besitzen, kalibriert werden. Diese definierten Lösungen werden im folgenden als Standardlösungen bezeichnet. Weitere gebräuchliche Bezeichnungen für Standardlösungen sind Kalibratoren, Eichstandards oder Eichlösungen.

Die wichtigste Anforderung an Kalibratoren ist eine hohe Stabilität dieser Eichstandards. Zum einen muß die Richtigkeit des Tests und ein gutes Signal-Rausch-Verhältnis sichergestellt sein. Zum anderen muß eine zuverlässige Reproduzierbarkeit des Bestimmungsergebnisses über einen längeren Zeitraum gewährleistet sein. Viele Analyten, die als Kalibratoren eingesetzt werden, sind äußerst empfindlich gegenüber ihren Umgebungsbedingungen. Einen großen Einfluß auf die Stabilität der Kalibratoren besitzen die Temperatur, der pH-Wert und die Pufferbedingungen des Mediums, in dem sie sich befinden. Bei ungünstigen Bedingungen besteht die Gefahr der Hydrolyse, Proteolyse oder Denaturierung des Kalibrators. Der Einsatz eines nicht mehr intakten Kalibrators würde zu Fehlkalibrationen und -messungen führen.

Aus dem Stand der Technik sind verschiedene Methoden zur Stabilisierung von biologischen Makromolekülen (Analyten) bekannt.
So ist die Auswahl des richtigen Puffersalzes und pH-Werts wichtig, aber auch der Zusatz von Hilfsstoffen wie Detergenzien, Proteinen, Zuckern, Salzen etc. sind in der einschlägigen Literatur beschrieben (zur Übersicht: Shami et al., Tibtech 1989, Vol. 7, S. 186-190).

So wird in der WO 87/00196 eine Methode zum Schutz vor Denaturierung während des Trocknens von Proteinen und anderen Makromolekülen beschrieben. Zur Vermeidung von Denaturierung findet der Trocknungsvorgang in Gegenwart von Trehalose oder ähnlichen Zuckern statt.

In der WO 91/18091 wird eine Methode zur schonenden Aufbewahrung von Enzymen und anderen biologischen Makromolekülen offenbart. Durch den Zusatz von bestimmten nicht-reduzierenden Zuckern bzw. Zuckerderivaten erhalten die so behandelten Enzyme eine höhere Temperaturstabilität. Außerdem wird eine größere Stabilität bei der Aufbewahrung im trockenen Zustand erzielt. Der Einsatz hoher Zuckerkonzentrationen in Eichstandards ist keine geeignete Stabilisierungsmethode. Zuckerhaltige Lösungen weisen oft eine hohe Viskosität auf, so daß beim Pipettieren insbesondere kleiner Volumina Fehler auftreten können. Durch Verdünnen könnte die Zuckerkonzentration gesenkt werden, wobei die Verdünnungsschritte wiederum eine Fehlerquelle darstellen, so daß die Konzentration des Eichstandards verfälscht werden würde.

Die Stabilisierung von Proteinen kann auch dadurch erfolgen, daß ein organisches Polymer mit dem zu schützenden Protein, wie in der EP 0 513 332 offenbart, kovalent verknüpft wird, also ein Konjugat aus organischem Polymer und dem zu stabilisierenden Protein gebildet wird. Die so hergestellten Konjugate weisen eine hohe Stabilität gegenüber hohen Temperaturen, organischen Lösungsmitteln und proteolytischen Angriffen durch Enzyme auf. Allerdings wird hierbei die natürliche Struktur des Proteins stark verändert, was seine Funktion als Kalibrator beeinflussen kann.

In der EP 0 170 983 wird beschrieben, daß durch den Zusatz von hydrolysiertem Ovalbumin besonders monoklonale Antikörper gegen thermische Denaturierung geschützt werden können.

Der Zusatz von nicht genau definierten Proteinhydrolysaten kann die Eigenschaften des Kalibrators beeinflussen.

Im Stand der Technik wird auch die stabilisierende Wirkung von spezifisch bindenden Antikörpern auf Enzyme beschrieben.
So bewirkt beispielsweise die Bindung von monoklonalen Antikörpern an alkalische Phosphatase, daß dieses Enzym nicht mehr von der Protease Trypsin gespalten werden kann (Jemmerson und Stigbrand. Febs Letters 1984, Vol. 173, S. 357-359),

Auch die Stabilisierung von Enzymen gegenüber thermischer Denaturierung durch die Bindung spezifischer Antikörper ist bekannt. Beispiele hierfür sind die Enzyme β-Galactosidase (Melchers und Messer, Biochem. Biophys. Res. Comm. 1970, Vol. 40), Hexaminidase A (Ben-Yoseph et al. Immunochemistry 1974, Vol. 12, S. 221-226) und die saure Phosphatase PAP (prostatic acid phosphatase; Sawada et al. Chem Pharm. Bull. 1981, Vol. 29, S. 2934-2939). Bei diesen beispielhaft genannten Enzymen wird das Enzym mit dem spezifisch bindenden Antikörper inkubiert. Nachdem die Lösung erhitzt worden ist, wird die enzymatische Aktivität des Enzyms überprüft. Die Antikörper schützen in diesen Fällen also das aktive Zentrum des Enzyms.

Der Inhalt der Anmeldung EP-A-780 687 gilt gemäß Artikel 54(3) und (4) EPÜ als Stand der Technik. Diese Anmeldung beschreibt einen Kalibrator zur Verwendung in einem Sandwich-lmmunoassay bestehend aus einem Konjugat aus einem Antikörper und einem für den Analyten spezifischen Peptid oder Peptidderivat.

EP-A-650 053 beschreibt einen flüssig-stabilen Kalibrator zur Verwendung in einem Test zum Nachweis eines Analyten, welcher aus einem Konjugat aus mindestens zwei Bindungsstellen besteht, die spezifisch mit der analytspezifischen Binderegion des Rezeptors, der zum Nachweis im Test eingesetzt wird, binden, wobei die Bindungsstellen durch mindestens eine lösliche Trägersubstanz verbunden sind und das Konjugat das Produkt einer synthetischen oder rekombinanten Herstellung ist, und der in einer wäßrigen Lösung in einer genau bekannten Menge gelöst ist.

DE-C-42 29 591 beschreibt ein immunologisches Verfahren zur Bestimmung eines Analyten sowie eine Vorrichtung zur Durchführung des Verfahrens in denen ein Antikörper mit einem Tracer abgesättigt ist.

Im Stand der Technik war das Problem der mangelnden Langzeitstabilität von wässrigen Lösungen, die markierte Proteine, d.h. Konjugate enthalten, bekannt. Ein Verfahren zur Stabilisierung von hydrolyseempfindlichen Molekülen in Lösung wird in der EP 0 487 301 offenbart. Hierbei wird allerdings nur der Marker oder das Label, bei dem es sich um ein Enzym handelt, von einem spezifisch bindenden Antikörper gebunden und so stabilisiert. In einem Eichstandard muß jedoch primär der Analyt und nicht der Marker stabilisiert werden.

Die stabilisierende Wirkung der oben beschriebenen Methoden ist nicht für alle Analyten gleich groß. Es gibt eine große Anzahl von Analyten, die durch diese Methoden nicht in dem Maße stabilisiert werden können, wie es für die Verwendung in einer Standardlösung notwendig wäre.

Eine weitere gebräuchliche, dem Fachmann bekannte Methode zur Stabilisierung von hydrolyseempfindlichen Molekülen oder Analyten ist das Lyophilisieren des Analyten in einem optimierten Medium. Dies hat den Nachteil, daß das Lyophilisat vor der Benutzung reproduzierbar aufgelöst und der Analyt unter definierten Bedingungen rekonstituiert werden muß, was zeitaufwendig ist und mit Fehlern behaftet sein kann. Es besteht hierbei die Gefahr von Fehlern durch ungenaues Pipettieren der Rekonstitutionsflüssigkeit. Außerdem neigen lyophilisierte Produkte danach oft zur Trübungsbildung, wodurch Fehler in der klinischen Diagnostik, insbesondere bei kolorimetrischen Auswertungen, auftreten können. Diese Fehlerquellen sollten für Eichstandards möglichst ausgeschlossen werden, da sonst die gesamte Meßreihe fehlerhaft werden würde.

Aufgabe der vorliegenden Erfindung war es daher, ein Verfahren zur Herstellung von Standardlösungen bereitzustellen. Der in der Standardlösung vorhandene Analyt sollte über längere Zeit möglichst auch bei höheren Temperaturen stabil sein. Die Standardlösung sollte in flüssiger Form als gebrauchsfertige Lösung verfügbar sein.

Die erfindungsgemäße Lösung der Aufgabe besteht in der Bereitstellung eines Verfahrens zur Herstellung von Standardlösungen, die eine hohe Stabilität aufweisen. Das Verfahren ist dadurch gekennzeichnet, daß ein sich in einer Lösung befindlicher Analyt oder ein Analytanalogon mit einem Konjugat aus einem spezifischen Bindepartner für den Analyten und einer Markierung umgesetzt wird, so daß sich ein stabilisierter Komplex aus dem Analyten und dem Konjugat bildet. Der Analyt ist in einer genau bekannten Konzentration in der Lösung vorhanden.

Durch dieses Verfahren zur Stabilisierung von Kalibratoren wird vermieden, daß der Kalibrator als Lyophilisat vorliegen muß. Auch der Zusatz von fremden Stabilisatoren wird vermieden oder zumindest verringert. Die permanente Kühlung oder gar Eiskühlung der Standardlösung kann vermieden oder stark reduziert werden, was in der täglichen Laborpraxis einen großen praktischen Vorteil darstellt. Durch dieses Verfahren wird auch gewährleistet, daß der Kalibrator einer Erwärmung über einen längeren Zeitraum, wie sie zum Beispiel bei direkter Sonneneinstrahlung auf das Gefäß, das die Standardlösung enthält, vorkommt, standhält und stabil bleibt.

Der Begriff Analyt umfaßt spezifisch bindefähige Substanzen wie beispielsweise Antigene, Antikörper, Antikörperfragmente, Peptide, modifizierte Peptide, Proteine, modifizierte Proteine, Haptene, Hormone, Nukleinsäuren, Zucker und Analytanaloga bzw. Analytderivate, Bevorzugt ist der Analyt ein Antigen, Antikörper oder Antikörperfragment bzw. Analoga hiervon.

Als Bindepartner werden Substanzen verstanden, die an den Analyten spezifisch binden. Dazu gehören monoklonale und polyklonale Antikörper, Antikörperfragmente wie Fab-, Fab'- und F(ab)₂'-Fragmente, Rezeptoren, Enzyme, Lektine und Nukleinsäuren. Bevorzugte Bindepartner sind Antikörper und Antikörperfragmente oder, falls der Analyt selbst einen Antikörper darstellt, das zugehörige Antigen. Es wird mindestens ein Bindepartner eingesetzt, gegebenenfalls können auch zwei oder mehr Bindepartner an den Analyten binden.

Die Paare, die bei der Bindung des Analyten an den spezifischen Bindepartner entstehen, sind dem Fachmann bekannt. Geeignet sind beispielsweise folgende Paare: Antigen/Antikörper, Hapten/Antikörper, Biotin/Avidin, Biotin/Streptavidin, Hormon/Rezeptor, Protein/Antiprotein, Protein-A/Immunglobulin, Hämoglobin/Haptoglobin, Glykoprotein/Lektin, Enzym/Substrat oder Nukleinsäure/Nukleinsäure.

Das Konjugat besteht aus dem spezifischen Bindepartner für den Analyten und einer Markierung. Es können auch mehr als eine Markierung auf dem spezifischen Bindepartner vorhanden sein.
Alle gängigen, dem Fachmann bekannten Markierungen können Verwendung finden. Als Markierung kann beispielsweise Biotin, ein Hapten, ein Enzym, eine radioaktive Substanz, ein Isotop, eine fluoreszierende oder chemilumineszierende Substanz verwendet werden. Die Markierung dient einerseits dazu, das Konjugat nachzuweisen. Die Bestimmung wird nach einer dem Fachmann wohlbekannten Methoden durchgeführt. Zu diesen bevorzugten Markierungen gehören chemilumineszierende Substanzen. Andererseits kann die Bestimmung auch dazu dienen, das Konjugat an eine Festphase zu binden. Falls die Markierung aus Biotin besteht, würde eine Festphase verwendet werden, an die Avidin oder Streptavidin gebunden ist Bevorzugte Markierungen sind solche, die die Bindung an eine Festphase ermöglichen, wie beispielsweise Biotin.
Auch Latexpartikel und ähnliche Partikel, die in homogenen Immunoassays angewendet werden, sind als Markierung des Bindepartners geeignet. In diesem Fall erfolgt der Nachweis in dem Fachmann bekannter Weise durch Trübungsmessung. Die Markierung des Bindepartners kann auch auf indirekte Weise erfolgen. Das heißt, der Bindepartner (z.B. ein Antikörper) wird von einem weiteren Bindepartner (z.B. ein Anti-Antikörper), der selbst mit einer dem Fachmann bekannten Markierung versehen ist, spezifisch gebunden. Der Nachweis erfolgt dann über die Markierung des zweiten Bindepartners nach dem Fachmann bekannten Methoden. Der Vorteil einer solchen indirekten Markierung ist darin zu sehen, daß ein einziges Konjugat als Universalmarkierung bei mehreren Testen eingesetzt werden kann. Die Herstellung der Konjugate erfolgt in an sich bekannter Weise.

Ein Vorteil bei der Verwendung von Konjugaten ist, daß die Bindung des Analyten zu dem analytspezifischen Bindepartner oder den analytspezifischen Bindepartnern bereits vorliegt. Dies könnte eine Zeitersparnis bedeuten.

Ein weiterer Vorteil des Einsatzes eines Konjugates gegenüber einem nicht-markierten Bindepartner ist, daß der zur Stabilisierung des Analyten verwendete Bindepartner direkt ein Bestandteil des sich später ausbildenden und nachzuweisenden Komplexes ist. Daher ist es nicht notwendig, zur Stabilisierung des Analyten einen weiteren Bindepartner zu benutzen, der ein anderes Epitop als der oder die Bindepartner im sich ausbildenden Komplex erkennt. Der Einsatz eines solchen zusätzlichen Bindepartners ist oft nicht möglich oder unzweckmäßig, weil der schützende zusätzliche Bindepartner im nachzuweisenden Immunkomplex stört oder weil vor allem bei kleinen Analyten ein zweites oder drittes Epitop nicht existiert
Bei entsprechender Größe des Analyten können jedoch auch mehrere Konjugate an den Analyten gebunden sein. Die Bindepartner binden dann an räumlich voneinander getrennte Epitope des Analyten. Jeder spezifische Bindepartner kann dabei eine andere Markierung tragen.

Das Korzentrationsverhältnis von Analyt zu Bindepartner bzw. Konjugat sollte 1:100 bis 1:10 000 betragen. Der Bindepartner bzw. das Konjugat sollte also in großem Überschuß in der Standardlösung vorhanden sein, damit für ausreichende Stabilität des Analyten gesorgt ist.

Die Standardlösung kann bei allen Testen eingesetzt werden, bei denen eine Bindung zwischen einem Analyten und einem Konjugat aus einem Bindepartner und einer Markierung stattfindet und die bereits vollzogene Bindung des Analyten an das Konjugat nicht die Testführung beeinträchtigt.

Zu den umfaßten Testführungen zählen heterogene Immunoassays wie der Sandwich-Test. Beim Sandwich-Test sind mindestens zwei spezifische Bindepartner notwendig Einer der spezifischen Bindepartner kann direkt oder indirekt an die Festphase gekoppelt sein. Bei direkter Kopplung eines Bindepartners an die Festphase wird als Eichlösung der Komplex aus Analyt und markiertem Bindepartner eingesetzt. In diesem Fall bildet sich der ganze Sandwich direkt an der Festphase aus. Der Nachweis des Analyten erfolgt über die Markierung des Bindepartners aus der Eichlösung.

Bei indirekter Kopplung eines Bindepartners an die Festphase ist einer der Bindepartner mit einer Markierung wie z.B. Biotin versehen, die die Bindung an die Festphase (z.B. über Avidin oder Streptavidin) ermöglicht. Als Eichlösung kann dann entweder der Komplex aus Analyt und Konjugat aus Bindepartner und einer Markierung, die die Bindung an die Festphase vermittelt, oder der Komplex aus Analyt und Konjugat aus Bindepartner und einer Markierung mit deren Hilfe die Detektion erfolgt oder der Komplex aus Analyt und beiden Konjugaten eingesetzt werden.

Als Festphase können die dem Fachmann geläufigen Festphasen verwendet werden. Bevorzugt sind Kunststoffröhrchen, die nach dem Fachmann bekannten Verfahren mit Streptavidin oder Avidin beschichtet werden können. Weiterhin sind bevorzugt Mikropartikel, die ebenfalls nach dem Fachmann bekannten Verfahren mit Streptavidin oder Avidin beschichtet werden können.

Eine bevorzugte Testführung ist der Sandwichassay, bei dem die Kopplung eines Bindepartners an die Festphase über Streptavidin- bzw. Avidin/Biotin erfolgt. Dabei ist die Festphase nach dem Fachmann bekannten Methoden mit Streptavidin oder Avidin beschichtet. Ein spezifischer Bindepartner ist ebenfalls nach dem Fachmann wohlbekannten Methoden kovalent mit Biotin gekoppelt. Die wesentlichen Bestandteile der Eichlösung sind bei dieser Testführung bevorzugt ein Konjugat aus analytspezifischem Antikörper und Biotin. Der Analyt liegt als Komplex gebunden an das Konjugat vor.

Zu den weiteren bevorzugten Testführungen gehören homogene Testführungen wie turbidimetrische Tests. In diesem Fall könnte die Markierung beispielsweise aus Latexpartikeln bestehen. Mehrere Bindepartner können an eine Markierung gekoppelt sein. Der Analyt wird dann von den Bindepartnern gebunden, so daß der als Eichstandard eingesetzte Komplex entsteht. Durch Zusatz einer definierten Menge eines weiteren Bindepartners, der beispielsweise ein Antikörper ist, würde die Vernetzung der Konjugate aus Bindepartner und Latexpartikel über den Analyten erfolgen. Die auftretende Trübung wird anschließend bestimmt und stellt ein Maß für die Menge des vorhandenen Analyten dar.

In der Standardlösung können die üblichen dem Fachmann geläufigen Puffersubstanzen vorhanden sein, sowie Salze, Zucker, Detergenzien, Konservierungsmittel, Protease-Hemmer und RSA in Konzentrationsbereichen, die vom Fachmann leicht zu ermitteln sind.

Weiterhin betrifft die vorliegende Erfindung die Verwendung eines Konjugates aus einem spezifischen Bindepartner und einer Markierung zur Stabilisierung eines Analyten in Lösung.

Gegenstand der Erfindung ist außerdem eine Standardlösung bestehend aus einem Komplex aus Analyt und einem Konjugat aus einem spezifischen Bindepartner und einer Markierung.
Bevorzugte Bindepartner sind hierbei Antikörper, die den Analyten spezifisch binden. Eine bevorzugte Markierung ist Biotin. Eine weitere bevorzugte Markierung sind chemilumineszierende Substanzen. In dieser Eichlösung liegt der Analyt in bestimmten, genau bekannten und nur in sehr engen Grenzen variierenden Konzentrationen vor.

Ein weiterer Gegenstand der Anmeldung ist ein Verfahren zum Nachweis eines Analyten bestehend aus den Schritten
Umsetzen der den Analyten enthaltenden Probe mit einem Konjugat aus einem spezifischen Bindepartner für den Analyten und einer Markierung, über die gegebenenfalls die Bindung an eine Festphase erfolgt,
gegebenenfalls Umsetzen dieser Lösung mit einem weiteren Bindepartner oder Konjugat,
Detektion des in der Probe enthaltenen Analyten über das Signal der Markierung,
Vergleichen des so erhaltenen Signals mit einem Signal, das mit Hilfe der oben beschriebenen Eichlösung erhalten wird.

Die Inkubation der Testbestandteile kann gleichzeitig oder nacheinander erfolgen. Ebenso ist die Reihenfolge der Schritte nicht festgelegt. So kann zuerst die Kopplung eines Konjugats aus spezifischem Bindepartner und Markierung an die Festphase erfolgen, bevor die Bindung des Komplex bestehend aus Analyt und einem Konjugat bzw. der Eichlösung an den festphasengekoppelten Bindepartner erfolgt.

### Fig. 1:

Die Darstellung zeigt die Stabilisierung des Analyten LH in Eichstandards durch den Einsatz eines Konjugats aus einem monoklonalen Antikörper und Biotin. Die Belastung erfolgte bei verschiedenen Temperaturen jeweils über drei Wochen. Die Meßwerte sind in Tabelle 1 nochmals aufgeführt.

### Fig. 2:

Bei dieser Darstellung erfolgte die Belastung des LH-MAK-Biotin-Kalibrators bei konstant 35°C über verschiedene Zeiträume.
Die Meßwerte sind in Tabelle 2 nochmals aufgeführt.

Die folgenden Beispiele erläutern die Vorteile der vorliegenden Erfindung.

### Beispiel 1: Herstellung der Kalibratoren, nicht stabilisiert

### 1.1. Einsatzstoffe:

- humanes LH (Luteinisierendes Hormon),
   isoliert aus menschlicher Gehimanhangdrüse, lyophilisiert in 0,05 M NH₄HCO₃ pH 8,0 für Enzymun-Test® LH (Boehringer Mannheim GmbH, Deutschland, Ident.-Nr. 1 488 678); Bezugsquelle: UCB-Bioproducts S.A. - Chemin du Foriest, B-1420 Braine-l'Alleud, Belgien
- Humanserum, LH-frei,
   abgereichert durch Immunsorption und stabilisiert mit den Konservierungsmitteln N-Methylisothiazolon (MIT), Oxy-Pyrion und Phenyl-Methyl-Sulfonylfluorid (PMSF).

### 1.2. Lösen des LH, human:

50 µg LH werden mit einer Konzentration von c=25µg/ml mit Humanserum, LH-frei aufgenommen (Stocklösung I) und 30 - 60 Minuten bei 2 - 8°C rekonstituiert.

### 1.3. Verdünnen des LH, human:

Die Stocklösung I wird mit Humanserum, LH-frei in einem ersten Schritt auf eine Konzentration von c = 5 IU/ml (Stocklösung II) und in einem zweiten Schritt auf die Konzentrationen der in der folgenden Tabelle aufgeführten Arbeitslösungen verdünnt.

| Arbeitslösung | Konzentration |
|---|---|
| | [mlU/ml] |
| 1 | 2.5 |
| 2 | 5 |
| 3 | 10 |
| 4 | 20 |
| 5 | 40 |

### Beispiel 2: Herstellung der Kalibratoren, stabilisiert:

### 2.1. Einsatzstoffe:

- humanes LH, wie in Beispiel 1 beschrieben
- Humanserum, LH-frei, wie in Beispiel 1 beschrieben
- MAK<LH>-Bi-Konjugat.
   biotinylierter monoklonaler Antikörper gegen LH (Boehringer Mannheim GmbH, Deutschland. Ident,-Nr. 1 547 038).
   5 mg/ml MAK <LH>-Bi in 25 mM Kaliumphosphat, 100 mM NaCl, 1% Rinderserumalbumin, pH 7.0

### 2.2. Herstellen der stabilisierenden Matrix

Das Humanserum, LH-frei wird mit der obigen Lösung von MAK<LH>-Bi-Konjugat in dem Verhältnis gemischt, daß die Soll-Konzentration erreicht wird. So wurde z.B. bei einer Soll-Konzentration von 20µg/ml 1 Liter Humanserum, LH-frei mit 4 ml der Lösung von MAK<LH>-Bi-Konjugat gemischt. Die Mischung wurde durch Rollen 30 Minuten homogenisiert.

### 2.3. Lösen des LH, human

50 µg LH werden mit einer Konzentration von c = 25 µg/ml mit Humanserum, LH-frei, aufgenommen (Stocklösung 1) und 30 - 60 Minuten bei 2 - 8°C rekonstituiert.

### 2.4. Verdünnen des LH. human

Die Stocklösung 1 wird mit stabilisierender Matrix in einem ersten Schritt auf eine Konzentration von c = 5 IU/ml (Stocklösung II) und in einem zweiten Schritt auf die Konzentrationen der in der folgenden Tabelle aufgeführten Arbeitslösungen verdünnt.

| Arbeitslösung | Konzentration |
|---|---|
| | [mlU/ml] |
| 1 | 2.5 |
| 2 | 5 |
| 3 | 10 |
| 4 | 20 |
| 5 | 40 |

### Beispiel 3: Belasten der Kalibratoren und Vermessen der Arbeitslösungen

### 3.1. Belasten der Kalibratoren

Die Arbeitslösungen werden in 1ml-Portionen in verschließbaren Polyethylengefäße gefüllt und diese bei den angegebenen Temperaturen für die angegebenen Zeiträume gelagert.

### 3.2. Vermessen der Arbeitslösungen

Die Arbeitslösungen werden mit dem ElecSys®LH Immunoassay, Boehringer Mannheim GmbH, Deutschland nach Herstellerangaben vermessen.
Es wird nach dem Sandwichprinzip verfahren. Zur erfindungsgemäßen Standardlösung wird als weiterer spezifischer Bindepartner ein Antikörper gegeben, der mit einem Ruthenium-Komplex markiert ist. Der Nachweis erfolgt über ein vom Ruthenium-Komplex ausgesandtes Chemilumineszenz-Signal.

Die Ergebnisse sind in den Tabellen 1 und 2 dargestellt.

**Tabelle 1:**

| Stabilisierung des LH-Kalibrators durch Zusatz von MAK-Bi (Graphik s. Fig.1) | | | | | |
|---|---|---|---|---|---|
| Belastung über 3 Wochen bei verschiedenen Temperaturen | | | | | |
| | **Belastungstemperatur** | | | | |
| | **5°C** | **10°** | **16°C** | **22°C** | **35°C** |
| | Analyt-Wiederfindung gegenüber der Anfangsmessung [%] | | | | |
| nicht stabilisiert | 98 | 100 | 97 | 82 | 22 |
| stabilisiert mit 2 µg/ml | 98 | 103 | 100 | 96 | 77 |
| stabilisiert mit 20 µg/ml | 98 | 104 | 102 | 102 | 96 |

**Tabelle 2:**

| Stabilisierung des LH-Kalibrators durch Zusatz von MAK-Bi (Graphik s. Fig. 2) | | |
|---|---|---|
| Belastung bei 35°C über unterschiedliche Zeiträume | | |
| Belastungsdauer [Wochen] | Analyt-Wiederfindung im ELISA nach Belastung [%] | |
| | nicht stabilisiert | stabilisiert |
| 0 | 100 | 100 |
| 1 | 49 | 95 |
| 3 | 32 | 92 |
| 6 | 12 | 90 |

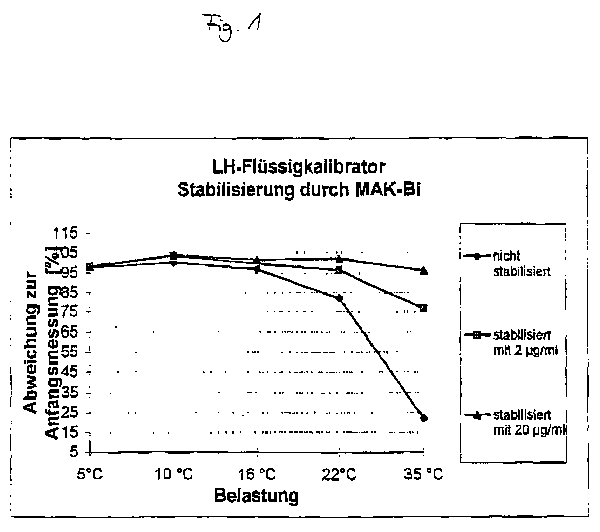

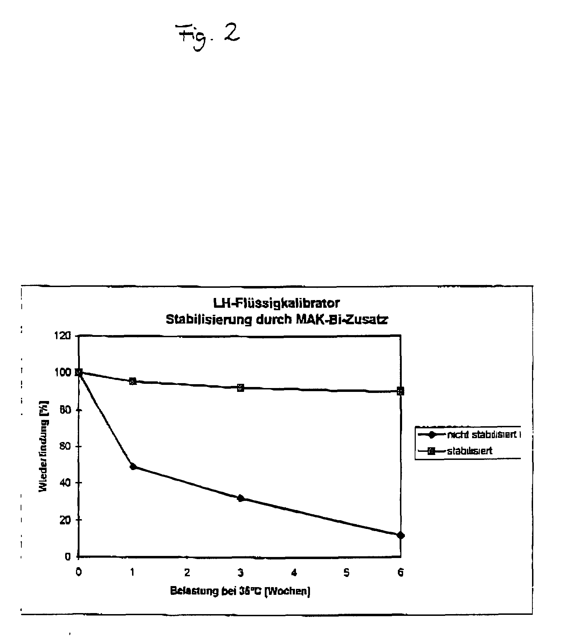

## Patentansprüche

1. Verfahren zur Herstellung von Standardlösungen, **dadurch gekennzeichnet, daß** ein sich in einer Lösung befindlicher Analyt mit einem Konjugat aus einem spezifischen Bindepartner für den Analyten und einer Markierung umgesetzt wird, so daß sich ein stabilisierter Komplex aus dem Analyten und dem Konjugat bildet.

2. Verfahren nach Anspruch 1, **dadurch gekennzeichnet, daß** der spezifische Bindepartner ein Antikörper oder Antikörperfragment ist.

3. Verfahren nach Anspruch 1 oder 2, **dadurch gekennzeichnet, daß** die Markierung die Bindung des Konjugates an eine Festphase ermöglicht.

4. Verwendung eines Konjugates aus einem Bindepartner und einer Markierung zur Stabilisierung eines Analyten in Lösung.

5. Standardlösung bestehend aus einem Komplex aus Analyt und einem Konjugat aus einem Bindepartner und einer Markierung.

6. Verfahren zum Nachweis eines Analyten bestehend aus den Schritten
a) Umsetzen der den Analyten enthaltenden Probe mit einem Konjugat aus einem spezifischen Bindepartner für den Analyten und einer Markierung, über die gegebenenfalls die Bindung an eine Festphase erfolgt,
b) gegebenenfalls Umsetzen dieser Lösung mit einem weiteren Bindepartner oder Konjugat.
c) Detektion des in der Probe enthaltenen Analyten über das Signal der Markierung.
**dadurch gekennzeichnet, daß** das so erhaltene Signal mit einem Signal, das mit Hilfe der Standardlösung gemäß Anspruch 5 erhalten wird, verglichen wird.

7. Verfahren zum Nachweis eines Analyten nach Anspruch 6, **dadurch gekennzeichnet, daß** die Schritte a und b gleichzeitig erfolgen.

8. Verfahren zum Nachweis eines Analyten nach Anspruch 6, **dadurch gekennzeichnet, daß** die Schritte a und b nacheinander erfolgen.

## Claims

1. Process for the production of standard solutions, wherein an analyte present in a solution is reacted with a conjugate composed of a specific binding partner for the analyte and a label so that a stabilized complex forms comprising the analyte and the conjugate.

2. Process as claimed in claim 1, wherein the specific binding partner is an antibody or an antibody fragment.

3. Process as claimed in claim 1 or 2, wherein the label enables the conjugate to bind to a solid phase.

4. Use of a conjugate composed of a specific binding partner for an analyte and a label to stabilize the analyte in solution.

5. Standard solution composed of a complex comprising analyte and a conjugate of a specific binding partner for the analyte and a label.

6. Method for the detection of an analyte comprising the steps
a) Reacting the sample containing the analyte with a conjugate composed of a specific binding partner for the analyte and a label via which binding to a solid phase optionally takes place,
b) optionally reacting this solution with a further binding partner or conjugate,
c) detecting the analyte present in the sample by means of the signal of the label,
wherein the signal obtained in this manner is compared with a signal which is obtained with the aid of a standard solution as claimed in claim 5.

7. Method for the detection of an analyte as claimed in claim 6, wherein steps a and b are carried out simultaneously.

8. Method for the detection of an analyte as claimed in claim 6, wherein steps a and b are carried out in succession.

## Revendications

1. Procédé de préparation de solutions étalon, **caractérisé en ce qu'**un analyte se trouvant dans une solution est mis à réagir avec un conjugué issu d'un partenaire de liaison spécifique pour l'analyte et un marqueur, de sorte à former un complexe stabilisé issu de l'analyte et du conjugué.

2. Procédé selon la revendication 1, **caractérisé en ce que** le partenaire de liaison spécifique est un anticorps ou un fragment d'anticorps.

3. Procédé selon la revendication 1 ou 2, **caractérisé en ce que** le marqueur permet de lier le conjugué à une phase solide.

4. Utilisation d'un conjugué issu d'un partenaire de liaison spécifique à un analyte et d'un marqueur pour stabiliser l'analyte en solution.

5. Solution étalon constituée d'un complexe issu d'un analyte et d'un conjugué issu d'un partenaire de liaison spécifique à l'analyte et d'un marqueur.

6. Procédé pour détecter un analyte, comprenant les étapes de :
a) réaction de l'échantillon contenant l'analyte avec un conjugué issu d'un partenaire de liaison spécifique pour l'analyte et un marqueur par l'intermédiaire duquel la liaison se réalise éventuellement à une phase solide,
b) éventuellement la réaction de cette solution avec un autre partenaire de liaison ou conjugué,
c) détection de l'analyte contenu dans l'échantillon par l'intermédiaire du signal du marqueur,
**caractérisé en ce que** le signal ainsi obtenu est comparé avec un signal qui est obtenu à l'aide de la solution étalon selon la revendication 5.

7. Procédé pour détecter un analyte selon la revendication 6, **caractérisé en ce que** les étapes a et b se réalisent en même temps.

8. Procédé pour détecter un analyte selon la revendication 6, **caractérisé en ce que** les étapes a et b se réalisent l'une après l'autre.
